# EUROPEAN PATENT SPECIFICATION

(11) **EP 2 866 025 B1**
(45) Date of publication and mention of the grant of the patent: **16.08.2023**
(21) Application number: 13808761.4
(22) Date of filing: 25.06.2013
(51) Int. Cl.: G01N 27/327, G01N 27/416, C12Q 1/00

(54) **ENZYME ELECTRODE**
ENZYMELEKTRODE
ÉLECTRODE ENZYMATIQUE

(30) Priority: 25.06.2012 JP 2012142375
(43) Date of publication of application: 29.04.2015
(73) Proprietor: ARKRAY, Inc., Kyoto-shi, Kyoto 601-8045 (JP)
(72) Inventor: SEKIMOTO, Shinjirou, Kyoto-shi Kyoto 602-0008 (JP)
(74) Representative: Dehns
(86) International application number: PCT/JP2013/067381
(87) International publication number: WO 2014/002998

(56) References cited:
- WO-A1-97/22715
- WO-A1-2009/153777
- JP-A- 2001 516 039
- JP-A- 2001 520 367
- US-A1- 2010 261 072
- YU ET AL: "Development of amperometric glucose biosensor through immobilizing enzyme in a Pt nanoparticles/mesoporous carbon matrix", TALANTA, ELSEVIER, AMSTERDAM, NL, vol. 74, no. 5, 18 January 2008 (2008-01-18), pages 1586-1591, XP022426382, ISSN: 0039-9140, DOI: 10.1016/J.TALANTA.2007.10.005
- Anonymous: "APR-200, 500 Series (Oxazoline functional crosslinking agent)", , 1 June 2011 (2011-06-01), XP055248402, Retrieved from the Internet: URL:http://www.ghenmaterials.com/uploads/8 /3/2/6/8326000/apr-oxazoline_tds.pdf [retrieved on 2016-02-08]
- Nippon Shokubai Co. ET AL: "EPOCROS: Novel Low toxic Polymer Crosslinker and Adjesion Promoting agent for waterborne", , 1 October 2016 (2016-10-01), XP055607681, Retrieved from the Internet: URL:https://www.shokubai.co.jp/en/products /functionality/pdf/epocros_e.pdf [retrieved on 2019-07-22]
- Tacey X. Viegas ET AL: "Polyoxazoline: Chemistry, Properties, and Applications in Drug Delivery", Bioconjugate Chemistry, vol. 22, no. 5, 18 May 2011 (2011-05-18), pages 976-986, XP055082338, ISSN: 1043-1802, DOI: 10.1021/bc200049d

## Description

### Technical Field

The present invention relates to an enzyme electrode.

### Background Art

There is known an enzyme electrode including an electrode as a base material and a detection layer in which an enzyme and conductive particles are immobilized on the surface of the electrode using a crosslinking agent or a binder. The enzyme electrode has a structure that takes electrons generated by an enzyme reaction out of the electrode.

Yu et al. (Talanta, 2008, Vol. 74, p1586-1591) describes enzyme electrodes for detecting glucose using Pt nanoparticles in a carbon matrix and the enzyme is crosslinked to gelatin which is attached to the electrode. US2010/0261072 describes enzyme electrodes with carbon particles and in which the enzyme may be attached to a sulfonated polymer. Other crosslinking agents are known such as oxazoline functional crosslinking agents (APR-200, 500 series). The properties and applications of polyoxazoline polymers are known (Viegas et al., 2011, Bioconjugate Chem., Vol. 22, p976-986).

### Prior Art Documents

### Patent Document

Patent Document 1: Japanese Laid-Open Patent Publication No. 63-222256
Patent Document 2: Japanese Laid-Open Patent Publication No. 2-99849
Patent Document 3: International publication No. WO 2007/055100
Patent Document 4: International publication No. WO 2009/037838
Patent Document 5: Japanese Laid-Open Patent Publication No. 2004-317421
Patent Document 6: Japanese Laid-Open Patent Publication No. 2007-222786
Patent Document7: U.S. Patent No. 6770729

### Summary of Invention

### Problems to be solved by the Invention

Generally, in order to properly hold an enzyme and conductive particles which are contained in the detection layer and to secure proper adhesion of the detection layer to a base material, it is conceived to increase the concentration of a crosslinking agent or a binder. However, when the concentration of the crosslinking agent or the binder is simply increased, the formation of a good electron transport pathway in the detection layer is prevented, and the response sensitivity of the enzyme electrode tends to decrease in proportion to the concentration of the crosslinking agent or the binder.

Further, in recent years, there has been a tendency that an enzyme electrode is reduced in size, and an enzyme electrode that maintains a proper immobilization state of a detection layer to a base material with a small amount of binder and also has good response sensitivity and reproducibility has been desired.

One aspect of the present invention has been made in view of the above circumstances, and is directed to provide an enzyme electrode having a detection layer which is properly immobilized to a base material and can obtain proper response sensitivity.

### Means for solving the Problems

In order to achieve an object described above, the one aspect of the present invention applies configurations below. Namely, the one aspect of the present invention is an enzyme electrode. The enzyme electrode includes an electrode (1) and a detection layer (2), the detection layer including an enzyme (5), conductive particles (6), and a non-conductive polymer, the non-conductive polymer being linked to i) at least a portion of the enzyme (5) by a linking chain containing nitrogen or oxygen and ii) at least a portion of the conductive particles (6) by a linking chain containing nitrogen or oxygen, wherein the linking chain is an amide ester bond, the non-conductive polymer contains an oxazoline group (7), the amide ester bond is derived from the oxazoline group and the conductive particles are carbon fine particles. The non-conductive polymer has a region of positive correlation between a concentration of the non-conductive polymer and a response sensitivity of the enzyme electrode.

The linking chain contains nitrogen (N) or oxygen (O) is e.g. a covalent bond. In the invention, the covalent bond is an amide ester bond. The amide ester bond is derived from the oxazoline group in the non-conductive polymer. The oxazoline group in the one aspect may be obtained in an amount of 4.5 mmol/g based on the non-conductive polymer.

The conductive particles are carbon fine particles. An average particle size of the conductive particles may be 100 nm or less. A specific surface area of the conductive particles may be 200 m²/g or more. The conductive particles contain a carboxyl group. Further, the conductive particles may include one or both of fine particle types selected from carbon nanotube and fullerene.

In the one aspect, a weight ratio among the enzyme, the conductive particles, and the non-conductive polymer may be 0.023 to 2.0:0.1 to 1.0:2.5 to 10.0.

One of other aspects of the present invention is an apparatus including the enzyme electrode (10) of the invention and a feed section configured to supply, to a load, a current generated by an enzyme reaction by energization to the enzyme electrode.

One of other aspects of the present invention is a method of producing the enzyme electrode (10) of the invention, comprising the steps of: preparing a reagent solution containing the enzyme (5), the conductive particles (6), and the non-conductive polymer; applying the reagent solution to an electrode (1); and drying the applied reagent solution to thereby form the detection layer on the electrode. A concentration of the non-conductive polymer added during the preparation of the reagent solution is 0.25% to 10%.

### Effects of the invention

The present invention provides an enzyme electrode as described hereinbefore having a detection layer which is properly immobilized to a base material and can obtain proper response sensitivity.

### Brief Description of Drawings

[Fig. 1] Fig. 1 is a schematic view illustrating the side surface or cross section of an enzyme electrode according to an embodiment.
[Fig. 2] Fig. 2 schematically illustrates the state in a detection layer 2 illustrated in Fig. 1.
[Fig. 3] Fig. 3 is a graph illustrating the relationship between the adhesion of a detection layer to an electrode and response sensitivity.

### Description of Embodiments

Hereinafter, an enzyme electrode as an embodiment of the present invention will be described with reference to drawings. Embodiments described below are for illustration purposes, and the present invention is not limited to the constitution of the following embodiments. The present invention is defined in the appended claims.

### [Constitution of enzyme electrode]

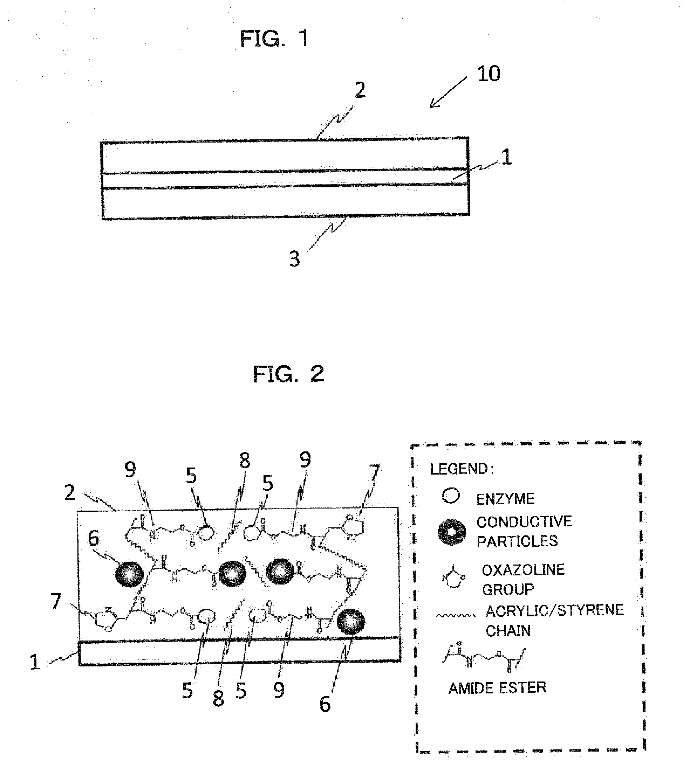

Fig. 1 is a schematic view illustrating the side surface or cross section of an enzyme electrode according to the embodiment. In Fig. 1, an enzyme electrode 10 includes an electrode (electrode layer) 1 and a detection layer 2 formed on the surface (upper surface in Fig. 1) of the electrode 1.

### <Electrode>

The electrode 1 is formed of a metallic material such as gold (Au), platinum (Pt), silver (Ag), and palladium, or a carbon material such as carbon. The electrode 1 is formed, for example, on an insulating substrate 3 as illustrated in Fig. 1. The substrate 3 is formed of an insulating material selected from various resins (plastics) including a thermoplastic resin, such as polyether imide (PEI), polyethylene terephthalate (PET), or polyethylene (PE), a polyimide resin, and an epoxy resin; glass; ceramics; and paper. Any known material can be applied as an electrode material forming the electrode 1 and a material for the insulating substrate 3. The size and the thickness of the electrode 1 and the insulating substrate 3 can be suitably set. Hereinafter, the combination of the insulating substrate 3 and the electrode 1 may be called a "base material."

### <Detection layer>

Fig. 2 schematically illustrates the state in the detection layer 2 illustrated in Fig. 1. As illustrated in Fig. 2, in the detection layer 2, the enzyme 5 and the conductive particles 6 are immobilized by a polymer containing an oxazoline group. Specifically, the enzyme 5, the conductive particles 6, and the oxazoline group 7, and an acrylic/styrene chain 8 are distributed in the detection layer 2, and an amide ester bond is formed by ring breakage of the oxazoline group 7 and a reaction with a carboxyl group of the enzyme 5 or the conductive particles 6.

That is, the detection layer 2 contains enzymes (enzyme molecules) 5, a plurality of conductive particles 6, and a plurality of non-conductive polymers (amide esters 9) that form an amide ester bond with at least a portion of enzymes 5 and at least a portion of the plurality of conductive particles 6.

The amide ester 9 is linked to the enzyme 5 or the conductive particles 6 to thereby result in a state where the enzyme 5 and the conductive particles 6 are strongly held or supported in the detection layer 2. Further, the amide ester bond leads to a state where the enzyme 5 and the conductive particles 6 are uniformly distributed in the detection layer 2. Thereby, a proper number of electron transport pathways are formed in which electrons generated by an enzyme reaction reach the electrode 1 through the conductive particles 6. Consequently, an improvement in the response sensitivity of an enzyme electrode is achieved. On the other hand, the detection layer 2 indicates proper adhesion to the electrode 1.

### <<Enzyme>>

The enzyme 5 is, for example, an oxidoreductase. For example, the enzyme 5 is glucose dehydrogenase (GDH). As the GDH, it is preferred to apply flavin adenine dinucleotide-dependent glucose dehydrogenase (FADGDH) which does not need an electron-transfer mediator. Further, an oxidoreductase containing a cytochrome can be applied as the enzyme 5. Examples include glucose dehydrogenase containing cytochrome (Cy-GDH), D-fructose dehydrogenase, and cellobiose dehydrogenase.

### <<Conductive particles>>

A higher-order structure formed from carbon is used as the conductive particles 6. In particular, the higher-order structure is one or more fine particle types (carbon fine particles) selected, for example, from conductive carbon black, Ketjen Black (registered trademark), carbon nanotube (CNT), and fullerene. Further, the conductive particles 6 contain at least a carboxyl group.

Note that the surface of the detection layer 2 may be covered with an outer-layer film made of cellulose acetate (CA) and the like.

### <<Weight ratio among enzyme, conductive particles, and binder and the like>>

The weight ratio among an enzyme, conductive particles, and a binder which constitute the detection layer 2 is preferably, for example, enzyme:conductive particles:binder = (0.023 to 2.0):(0.1 to 1.0):(2.5 to 10.0). Further, the conductive particles have an average particle size of, for example, 100 [nm] or less and a specific surface area of preferably, for example, 200 [m²/g] or more.

### [Method for preparing enzyme electrode]

Hereinafter, the enzyme electrode 10 as described above is prepared, for example, as follows. Specifically, a metal layer which functions as an electrode 1 is formed on one side of an insulating substrate 3. For example, a metal layer having a desired thickness (for example, about 30 nm) is formed by depositing a metallic material by physical vapor deposition (PVD, for example, sputtering) or chemical vapor deposition (CVD) on one side of an insulating substrate 3 in a film form having a predetermined thickness (for example, about 100 µm). An electrode layer formed of a carbon material can also be formed instead of the metal layer.

Next, a detection layer 2 is formed on an electrode 1. Specifically, an electrode solution containing conductive particles 6, an enzyme 5, and a non-conductive high polymer containing an oxazoline group as a binder is prepared. The electrode solution is dropped on the surface of the electrode 1. The electrode solution is dried and solidified on the electrode 1, thereby capable of obtaining the enzyme electrode 10 in which the detection layer 2 is formed on the electrode 1.

### [Examples]

Hereinafter, Examples of the enzyme electrode will be described.

### <Formulation of detection layer>

First, electrode solutions (reagent solutions) according to Example 1, Example 2, and Comparative Example each containing conductive particles, an enzyme, and a binder were prepared. In each of Example 1, Example 2, and Comparative Example, Ketjen Black (registered trademark, hereinafter represented by "KJB") was used as the conductive particles, and Cy-GDH was used as the enzyme.

On the other hand, in Example 1, EPOCROS ((registered trademark), hereinafter represented by "EPC") which is one of highpolymers containing an oxazoline group which is a covalent linking chain containing nitrogen (N) or oxygen (O) was used as a binder. In Example 2, poly-(2-ethyl-2-oxazoline) (hereinafter represented by "PEO") which is one of highpolymers containing an oxazoline group which is a covalent linking chain containing nitrogen (N) or oxygen (O) was used as a binder. Further, in Comparative Example, VYLONAL (registered trademark) was used as a binder. The final concentration of the electrode solution in Example 1, Example 2, and Comparative Example is as follows. The high polymer containing an oxazoline group which is a covalent linking chain containing nitrogen (N) or oxygen (O) is an example of "non-conductive polymers."

### <<Formulation in Example 1»

- KJB: 0.4 wt%
- Enzyme (Cy-GDH): 3.5 mg/mL
- Sodium phosphate buffer: 10 mM, pH 7
- EPC (EPOCROS WS-700, molecular weight: 40,000, manufactured by The Dow Chemical Company (NIPPON SHOKUBAI CO., LTD.))

As Example 1, four types of electrode solutions (samples) each having an EPC concentration of 0.25 [wt%], 0.5 [wt%], 1.0 [wt%], or 3.0 [wt%] were prepared.

### <<Formulation in Example 2»

- KJB: 0.4 wt%
- Enzyme (Cy-GDH): 3.5 mg/mL
- Sodium phosphate buffer: 10 mM, pH 7
- PEO (molecular weight; 50,000, an oxazoline-containing polymer, manufactured by Sigma-Aldrich Co.)

As Example 2, four types of electrode solutions (samples) each having a PEO concentration of 0.25 [wt%], 0.5 [wt%], 1.0 [wt%], or 3.0 [wt%] were prepared.

### <<Formulation in Comparative Example>>

- KJB: 0.4 wt%
- Enzyme (Cy-GDH): 3.5 mg/mL
- Sodium phosphate buffer: 10 mM, pH 7
- VYLONAL (registered trademark) MD-1200 (TOYOBO CO., LTD., a water-dispersed high-molecular weight copolyester resin", hereinafter represented by "MD-1200")

As Comparative Example, four types of electrode solutions (samples) each having an MD-1200 concentration of 0.25 [wt%], 0.5 [wt%], 1.0 [wt%], or 3.0 [wt%] were prepared.

Note that the KJB used in Example 1, Example 2, and Comparative Example had an average particle size of 100 [nm] or less and a specific surface area of 200 [m²/g] or more. Further, the proportion of the oxazoline group in the molecule in Example 1 was 4.5 [mmol/g].

### <A. Response sensitivity test>

### <<Preparation of enzyme electrode>>

A base material having a gold (Au) metal layer (electrode) formed by sputtering on the surface of a polyether imide (PEI) as an insulating substrate was prepared, and an electrode solution (a sample, a reagent solution) was dropped on the surface of the electrode under a condition of 10.0 µL/cm² per unit area (cm²) of the electrode. Then, the electrode solution on the surface of the electrode was air dried in an atmosphere of 23°C and 40% RH. In this way, an enzyme electrode was obtained in which a detection layer in which an enzyme and conductive particles were immobilized with each binder as described above was formed on the surface of the electrode. The detection layer contains a non-conductive polymer having a covalent linking chain containing nitrogen (N) or oxygen (O).

### <<Method for measuring response sensitivity>>

Next, an example of a method for measuring the glucose concentration using an enzyme electrode will be described. The response sensitivity to glucose was measured by chronoamperometry in which a potential difference of 400 mV is applied to an electrode system, as a measuring method.

First, a 0.1 M phosphate buffer (pH 7.0) was prepared as a measuring liquid, and kept at 37°C. Next, an enzyme electrode, in which a platinum electrode is used as a counter electrode and an Ag/AgCl electrode is used as a reference electrode, was electrically connected to a potentiostat and immersed in a 0.1 M phosphate buffer. Then, constant voltage (400 mV vs Ag/AgCl) was applied to a working electrode.

For the measurement of glucose concentration, a current value (nA) at a final glucose concentration of 0 mg/dL was first measured, and current values at 25 mg/dL, 50 mg/dL, 150 mg/dL, and 250 mg/dL were then continuously measured while continuously dropping a 2 M glucose solution. A current value corresponds to response sensitivity.

### <<Test results>>

The results of the measurement of glucose concentration in Example 1, Example 2, and Comparative Example are as described in the following Table 1, Table 2, and Table 3, respectively. The values in each Table represent response sensitivity (nA).

**[Table 1]**

| | EPC | | | |
|---|---|---|---|---|
| Glu (mg/dL) | 0.25% | 0.50% | 1% | 3% |
| 0 | 28.5 | 25.1 | 26.2 | 32.1 |
| 25 | 69.1 | 90.1 | 55.1 | 88.1 |
| 50 | 82 | 105.9 | 65.5 | 105.9 |
| 150 | 98.2 | 129.3 | 78.7 | 127.1 |
| 250 | 103.6 | 134.7 | 83.8 | 134.6 |

**[Table 2]**

| | PEO | | | | | |
|---|---|---|---|---|---|---|
| Glu (mg/dL) | 0.25% | 0.50% | 1% | 3% | 5% | 10% |
| 0 | 2.1 | 1.9 | 14.9 | 1.0 | 3 | 3.1 |
| 25 | 49.5 | 27 | 43.3 | 13.9 | 75.5 | 75.3 |
| 50 | 60.3 | 32.6 | 49.2 | 16.4 | 94.3 | 90.8 |
| 150 | 77.6 | 41.2 | 60.3 | 21.5 | 121 | 113 |
| 250 | 83.9 | 43.7 | 62.6 | 22.9 | 134 | 118 |

**[Table 3]**

| | MD-1200 | | | | | |
|---|---|---|---|---|---|---|
| Glu (mg/dL) | 0.25% | 0.50% | 1% | 3% | 5% | 7% |
| 0 | 1.4 | 0.9 | 1.8 | 2.4 | 1.4 | 1.6 |
| 25 | 25.4 | 20.4 | 16.5 | 14.7 | 16.4 | 10.5 |
| 50 | 29.5 | 24.3 | 19.5 | 16.4 | 19 | 12.1 |
| 150 | 34 | 29.5 | 23.2 | 19 | 22 | 14 |
| 250 | 35.3 | 31 | 24.2 | 19.7 | 22.8 | 14.1 |

According to the results of measurement described in Table 1, Table 2, and Table 3, in Example 1 and Example 2, it has been found that although a variation in measurement data is partly observed, positive correlation is observed in Examples when measurement data is seized as a whole, and there is a region where a retention or an improvement in response sensitivity is observed relative to an increase in the amount of a binder added. Specifically, Example 1 and Example 2 each have a region of positive correlation between the concentration of a binder (non-conductive polymer) added and the response sensitivity of an enzyme electrode. On the other hand, in Comparative Example, it has been found that although a variation in data is partly observed, negative correlation is observed when measurement data is seized as a whole, and there is a tendency that a reduction in response sensitivity is observed relative to an increase in the amount of a binder added. When a non-conductive polymer is added to an enzyme electrode, response sensitivity generally tends to be reduced as described in Comparative Example, but in Example 1 and Example 2, when a non-conductive polymer was added, an improvement in response sensitivity was observed. From Example 1 and Example 2, it has been found that when a high polymer containing an oxazoline group is used as a binder, an improvement in response sensitivity can be obtained irrespective of an increase in the amount of the binder added.

### <B. Adhesion (or strength) test method>

Next, enzyme electrodes prepared using the electrode solutions in Example 1, Example 2, and Comparative Example were each tested for the adhesion of the detection layer to the electrode (strength of the detection layer).

### <<Preparation of enzyme electrode>>

The same base material (base material in which an electrode is formed on an insulating substrate) as that used in the response sensitivity test was prepared, and each of the electrode solutions in Example 1, Example 2, and Comparative Example was dropped (applied) on the surface of an electrode under a condition of 10.0 µL/cm² per unit area (cm²) of the electrode. Then, the electrode solution on the surface of the electrode was air dried in an atmosphere of 23°C and 40% RH. In this way, a detection layer (enzyme electrode) formed on the electrode which is to be used for the adhesion (or strength) test was prepared.

### <<Adhesion (or strength) test>>

The adhesion of the detection layer to an electrode was tested according to JISK5600-5-6 Adhesion (Crosscut method).

### <<Test results>>

The test results by the crosscut method are described in Table 4 below. The test results are classified into 0 to 5, in which a numerical value of 0 represents the best adhesion, and a numerical value of 5 represents the poorest adhesion.

**[Table 4]**

| | EPC | PEO | MD 1200 |
|---|---|---|---|
| 0.25% | 4 | 4 | 4 |
| 0.50% | 3 | 4 | 4 |
| 1% | 2 | 2 | 3 |
| 3% | 1 | 1 | 1 |
| 5% | 1 | 1 | 3 |
| 7% | | | 4 |
| 10% | | 1 | |

From the above test results, in Example 1 and Example 2, it has been found that an improvement in adhesion is observed with an increase in the amount of a binder (high polymer containing an oxazoline group) added. On the other hand, in Comparative Example, it has been found that the relationship between the amount of a binder added and the adhesion is not observed, and the adhesion tends to be poor as a whole.

### <C. Relationship between response sensitivity test results and adhesion (or strength)>

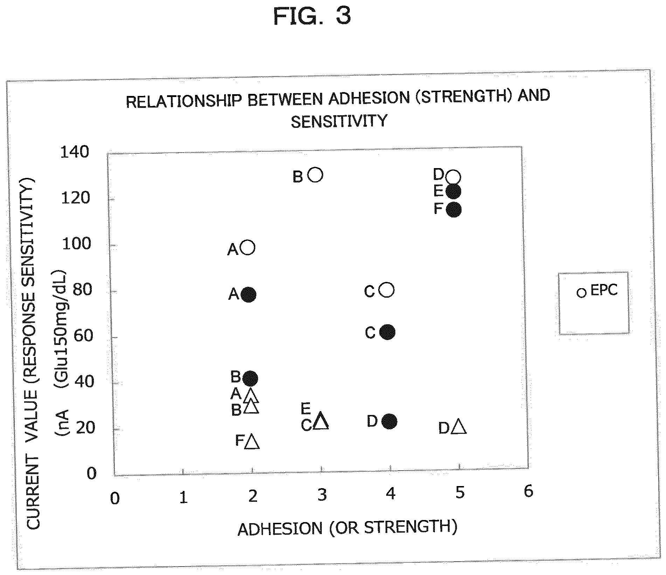

Fig. 3 is a graph illustrating the relationship between the results obtained in A. Response sensitivity test and the results obtained in B. Adhesion (or strength) test. In Fig. 3, white circle plots represent Example 1 (EPC), and the Roman alphabets A to D in the vicinity of the plots represent the concentration (amount added) of EPC. That is, A = 0.25%, B = 0.50%, C = 1%, and D = 3%.

Further, black circle plots in Fig. 3 represent Example 2 (PEO), and the Roman alphabets A to F in the vicinity of the plots represent the concentration (amount added) of PEG. That is, A = 0.25%, B = 0.50%, C = 1%, D = 3%, E = 5%, and F = 10%.

Further, triangular plots in Fig. 3 represent Comparative Example (MD-1200), and the Roman alphabets A to F in the vicinity of the plots represent the concentration (amount added) of MD-1200. That is, A = 0.25%, B = 0.50%, C = 1%, D = 3%, E = 5%, and F = 7%.

The axis of ordinates of the graph indicates a current value (response sensitivity) (nA) and represents test results when the glucose concentration is 150 mg/dL. On the other hand, the axis of abscissas of the graph represents adhesion (or strength). However, the numerical values in the graph represent the opposite of the numerical values for the classification used in the crosscut method described in Table 4. Specifically, a numerical value of 5 represents the best adhesion, and a numerical value of 0 represents the poorest adhesion.

As illustrated in Fig. 3, in Comparative Example, it has been found that even when the concentration of a binder is increased in order to enhance adhesion, the sensitivity tends to be hardly changed or to be reduced a little. On the other hand, in Example 1 and Example 2 (Examples in which a polymer containing an oxazoline group which is a covalent linking chain containing nitrogen (N) or oxygen (O) is applied as a binder), it has been found that response sensitivity is high compared with that in Comparative Example; a tendency of a reduction in sensitivity by increasing binder concentration is not observed; and a response sensitivity higher than that in Comparative Example can be maintained.

Thus, an enzyme electrode having high adhesion to a base material (electrode) and high response sensitivity, which has not been achieved by using an existing crosslinking agent and binder, can be prepared by preparing a detection layer containing a polymer containing an oxazoline group which is a covalent linking chain containing nitrogen (N) or oxygen (O). From Examples as described above, the concentration of the non-conductive polymer is, for example, 0.25% to 10%, preferably 0.5% to 5%, more preferably 1% to 3%.

### [Operations and effects of the embodiment]

According to the enzyme electrode 10 as described above, good response sensitivity can be obtained by immobilization of a proper enzyme 5 and conductive particles 6, and proper adhesion to a base material can be obtained. Particularly, since the concentration or the amount needed for proper immobilization can be reduced compared with an existing binder, it is expected to contribute to the reduction in size of the enzyme electrode 10.

Note that the enzyme electrode 10 can be applied, for example, to a biosensor which is applied to glucose measurement as described above, or an electronic apparatus (for example, measuring device). Alternatively, the enzyme electrode 10 can also be applied as a part of a power supply unit that supplies, to a load, response current generated by an enzyme reaction (current caused by electrons transferred to an electrode) as electric power through a feed section that connects the electrode and the load. The electronic equipment can include an electronic equipment including a biosensor to which the enzyme electrode according to the embodiment is applied and an electronic equipment including the above apparatus (power supply unit) to which the enzyme electrode according to the embodiment is applied.

### Description of the reference numerals

1: substrate (electrode), 2: detection layer, 3: insulating substrate, 5: enzyme, 6: conductive polymer, 7: oxazoline group, 8: acrylic/styrene chain, 9 amide ester

## Claims

1. An enzyme electrode (10), comprising:
an electrode (1); and
a detection layer (2), wherein:
the detection layer includes an enzyme (5), conductive particles (6), and a non-conductive polymer;
the non-conductive polymer is linked to i) at least a portion of the enzyme (5) by a linking chain containing nitrogen or oxygen and ii) at least a portion of the conductive particles (6) by a linking chain containing nitrogen or oxygen, wherein the linking chain is an amide ester bond, the non-conductive polymer contains an oxazoline group (7), the amide ester bond is derived from said oxazoline group and the conductive particles are carbon fine particles.

2. The enzyme electrode according to claim 1, wherein the oxazoline group is contained in an amount of 4.5 mmol/g based on the non-conductive polymer.

3. The enzyme electrode according to claim 1 or 2, wherein the average particle size of the conductive particles is 100 nm or less.

4. The enzyme electrode according to any one of claims 1 to 3, wherein the specific surface area of the conductive particles is 200 m²/g or more.

5. The enzyme electrode according to any one of claims 1 to 4, wherein the conductive particles include one or both of fine particle types selected from carbon nanotube and fullerene.

6. The enzyme electrode according to any one of claims 1 to 5, wherein the weight ratio among the enzyme, the conductive particles, and the non-conductive polymer is 0.023 to 2.0:0.1 to 1.0:2.5 to 10.0.

7. An apparatus, comprising:
an enzyme electrode (10) as defined in claim 1; and
a feed section configured to supply, to a load, a current generated by an enzyme reaction by energization to the enzyme electrode.

8. A method of producing an enzyme electrode (10) as defined in claim 1, comprising the steps of:
preparing a reagent solution containing the enzyme (5), the conductive particles (6), and the non-conductive polymer;
applying the reagent solution to an electrode (1); and
drying the applied reagent solution to thereby form the detection layer (2) on the electrode.

9. A method of producing the enzyme electrode according to claim 8, wherein the concentration of the non-conductive polymer added during the preparation of the reagent solution is 0.25% to 10%.

## Patentansprüche

1. Enzymelektrode (10), umfassend:
eine Elektrode (1); und
eine Nachweisschicht (2), wobei:
die Nachweisschicht ein Enzym (5), leitende Partikel (6) und ein nichtleitendes Polymer beinhaltet;
das nichtleitende Polymer mit i) mindestens einem Teil des Enzyms (5) durch eine Stickstoff oder Sauerstoff enthaltende Verbindungskette und ii) mindestens einem Teil der leitenden Partikel (6) durch eine Stickstoff oder Sauerstoff enthaltende Verbindungskette verbunden ist, wobei die Verbindungskette eine Amidesterbindung ist, das nichtleitende Polymer eine Oxazolingruppe (7) enthält, die Amidesterbindung von der Oxazolingruppe abgeleitet ist und die leitenden Partikel feine Kohlenstoffpartikel sind.

2. Enzymelektrode nach Anspruch 1, wobei die Oxazolingruppe in einer Menge von 4,5 mmol/g, bezogen auf das nichtleitende Polymer, enthalten ist.

3. Enzymelektrode nach Anspruch 1 oder 2, wobei die durchschnittliche Partikelgröße der leitenden Partikel 100 nm oder weniger beträgt.

4. Enzymelektrode nach einem der Ansprüche 1 bis 3, wobei die spezifische Oberfläche der leitenden Partikel 200 m²/g oder mehr beträgt.

5. Enzymelektrode nach einem der Ansprüche 1 bis 4, wobei die leitenden Partikel einen oder beide Feinpartikeltypen beinhalten, ausgewählt aus Kohlenstoffnanoröhren und Fulleren.

6. Enzymelektrode nach einem der Ansprüche 1 bis 5, wobei das Gewichtsverhältnis zwischen dem Enzym, den leitenden Partikeln und dem nichtleitenden Polymer 0,023 bis 2,0:0,1 bis 1,0:2,5 bis 10,0 beträgt.

7. Einrichtung, umfassend:
eine Enzymelektrode (10) wie in Anspruch 1 definiert; und
einen Speiseabschnitt, der so konfiguriert ist, dass er einer Last einen Strom zuführt, der durch eine Enzymreaktion durch Erregung der Enzymelektrode erzeugt wird.

8. Verfahren zur Herstellung einer Enzymelektrode (10) wie in Anspruch 1 definiert, das die folgenden Schritte umfasst:
Zubereiten einer Reagenzlösung, die das Enzym (5), die leitenden Partikel (6) und das nichtleitende Polymer enthält;
Auftragen der Reagenzlösung auf eine Elektrode (1); und
Trocknen der aufgetragenen Reagenzlösung, um dadurch die Nachweisschicht (2) auf der Elektrode zu bilden.

9. Verfahren zur Herstellung der Enzymelektrode nach Anspruch 8, wobei die Konzentration des nichtleitenden Polymers, das während der Herstellung der Reagenzlösung zugegeben wird, 0,25 % bis 10 % beträgt.

## Revendications

1. Électrode à enzyme (10), comprenant :
une électrode (1) ; et
une couche de détection (2), dans laquelle :
la couche de détection inclut une enzyme (5), des particules conductrices (6) et un polymère non conducteur ;
le polymère non conducteur est lié à i) au moins une partie de l'enzyme (5) par une chaîne de liaison contenant de l'azote ou de l'oxygène et ii) au moins une partie des particules conductrices (6) par une chaîne de liaison contenant de l'azote ou de l'oxygène, dans laquelle la chaîne de liaison est une liaison ester amide, le polymère non conducteur contient un groupe oxazoline (7), la liaison ester amide est dérivée dudit groupe oxazoline et les particules conductrices sont de fines particules de carbone.

2. Électrode à enzyme selon la revendication 1, dans laquelle le groupe oxazoline est présent à hauteur de 4,5 mmol/g sur la base du polymère non conducteur.

3. Électrode à enzyme selon la revendication 1 ou 2, dans laquelle la taille de particule moyenne des particules conductrices est de 100 nm ou moins.

4. Électrode à enzyme selon l'une quelconque des revendications 1 à 3, dans laquelle la surface spécifique des particules conductrices est de 200 m²/g ou plus.

5. Électrode à enzyme selon l'une quelconque des revendications 1 à 4, dans laquelle les particules conductrices incluent un ou les deux types de particules fines choisis parmi les nanotubes de carbone et le fullerène.

6. Électrode à enzyme selon l'une quelconque des revendications 1 à 5, dans laquelle le rapport pondéral entre l'enzyme, les particules conductrices et le polymère non conducteur est de 0,023 à 2,0:0,1 à 1,0:2,5 à 10,0.

7. Appareil, comprenant :
une électrode à enzyme (10) telle que définie dans la revendication 1; et
une section d'alimentation configurée pour fournir, à une charge, un courant généré par une réaction enzymatique par excitation à l'électrode à enzyme.

8. Procédé de production d'une électrode à enzyme (10) telle que définie dans la revendication 1, comprenant les étapes de :
préparation d'une solution de réactif contenant l'enzyme (5), les particules conductrices (6) et le polymère non conducteur ;
application de la solution de réactif à une électrode (1) ; et
séchage de la solution de réactif appliquée pour former ainsi la couche de détection (2) sur l'électrode.

9. Procédé de production de l'électrode à enzyme selon la revendication 8, dans lequel la concentration du polymère non conducteur ajouté pendant la préparation de la solution de réactif est de 0,25 % à 10 %.
